# EUROPEAN PATENT SPECIFICATION

(11) **EP 2 095 779 B1**
(45) Date of publication and mention of the grant of the patent: **08.01.2014**
(21) Application number: 09000969.7
(22) Date of filing: 23.01.2009
(51) Int. Cl.: A61B 17/32, A61B 18/14

(54) **Surgical treatment apparatus**
Chirurgische Behandlungsvorrichtung
Appareil de traitement chirurgical

(30) Priority: 26.02.2008 US 37441
(43) Date of publication of application: 02.09.2009
(73) Proprietor: Olympus Medical Systems Corp., Tokyo 151-0072 (JP)
(72) Inventor: Hideo, Sanai, Hachioji-shi Tokyo 192-8512 (JP)
(74) Representative: von Hellfeld, Axel

(56) References cited:
- DE-C2- 4 042 435
- JP-A- 2003 070 800
- JP-A- 2007 054 665

## Description

The present invention relates to a surgical treatment apparatus to treat a living tissue.

Various kinds of surgical treatment apparatuses to treat a living tissue are utilized. Jpn. Pat. Appln. KOKAI Publication No. 2007-54665, No. 2003-70800, and No. 2006-500116 disclose surgical treatment apparatuses using an ultrasonic vibration and a high-frequency current to treat a living tissue. In these surgical treatment apparatuses, a sheath and a handpiece are provided from a distal-end side to a proximal-end side. The handpiece accommodates a vibrator, and a probe is coupled with the vibrator. The probe is inserted through the sheath to be protruded from a distal-end portion of the sheath. Further, the probe is configured so that a high-frequency current flows through the probe. The probe being ultrasonic-vibrated by the vibrator or the probe through which the high-frequency current is flowing is pressed on the living tissue, thereby treating the living tissue. Furthermore, in each of the surgical treatment apparatuses disclosed in Jpn. Pat. Appln. KOKAI Publication No. 2007-54685 and No. 2003-70800, a switch portion to operate the surgical treatment apparatus is detachably arranged on the handpiece and in the surgical treatment apparatus disclosed in Jpn. Pat. Appln. KOKAI Publication No. 2006-500116, a switch portion is integrally arranged on the handpiece.

Like the surgical treatment apparatuses in Jpn. Pat. Appln. KOKAI Publication No. 2007-54665 , No. 2003-70800 and No. 2006-500116 , when arranging the switch portion on the handpiece, a diameter of the handpiece is usually increased, and the handpiece is hard to be held and operated.

In view of the above-explained problem, it is an object of the present invention to provide a handpiece easy to be held and operated.

In an aspect of the present invention, a handpiece according to claim 1 and a surgical treatment apparatus according to claim 4 are provided. The handpiece is configured to be held and operated by an operator and extended in an axial direction, wherein the handpiece includes: an operating portion to operate the surgical treatment apparatus; and a vibrating portion configured to output ultrasonic vibration to treat the living tissue, extended in the axial direction, including a distal end and a proximal end, and having an axial length being a length corresponding to one wavelength of the ultrasonic vibration, the vibrating portion includes: a vibration generating portion to generate the ultrasonic vibration, formed on a proximal-end side of the vibrating portion, and having an axial length being a length corresponding to a quarter wavelength of the ultrasonic vibration; and a vibration transmitting portion to transmit the ultrasonic vibration generated by the vibration generating portion, formed on a distal-end side of the vibrating portion, and having an axial length being a length corresponding to a three-quarter wavelength of the ultrasonic vibration, the vibration transmitting portion includes: a reduced diameter portion arranged on the proximal-end side of the vibration transmitting portion and having an outside diameter reduced from a proximal-end side toward a distal-end side; and an extending portion.

In the surgical apparatus according to this aspect, the axial length of the vibrating portion is the length corresponding to one wavelength of ultrasonic vibration, and so an axial length of the handpiece is a length corresponding to one wavelength of the ultrasonic vibration, and therefore an axial length that facilitates holding and operating is secured with respect to the handpiece. Furthermore, the axial length of the vibration generating portion is the length corresponding to the quarter wavelength of the ultrasonic vibration, and so an axial length of the portion of the handpiece where the operating portion is not arranged is a length corresponding to a quarter wavelength of the ultrasonic vibration. The axial length of the vibration transmitting portion is the length corresponding to the three-quarter wavelength of the ultrasonic vibration, and so an axial length of the operating portion is a length corresponding to a three-quarter wavelength of the ultrasonic vibration. Therefore, when the handpiece is held, the operating portion can be operated by a finger of a hand holding the handpiece in a state where the portion of the handpiece where the operating portion is not arranged is supported by a base of a thumb and an index finger. Moreover, since the outside diameter of the extending portion is smaller than the outside diameter of the vibration generating portion and the operating portion is arranged in the accommodation space on the radially outside of the extending potion, the outside diameter of the handpiece on the operating portion can be set to be equal to or smaller than the outside diameter of the handpiece on the vibration generating portion, and a diameter of the periphery of the operating portion is narrowed, thereby facilitating holding and operating the handpiece with respect to an entire balance.

In a preferred aspect of the present invention, the surgical apparatus is characterized in that the axial length of the vibrating portion is a length corresponding to one wavelength of the ultrasonic vibration.

In the surgical apparatus according to this aspect, since the axial length of the vibrating portion is a length corresponding to one wavelength of the ultrasonic vibration and so an axial length of the handpiece is a length corresponding to approximately one wavelength of the ultrasonic vibration, the miniaturized handpiece very easy to be held and operated is realized.

In a preferred aspect of the present invention, the surgical apparatus is characterized in that the operating portion includes two or more switches aligned in the axial direction.

In the surgical apparatus according to this aspect, although the two or more switches are aligned in the axial direction in the operating portion, since the sufficient axial length of the operating portion is secured, the two or more switches can be arranged with an appropriate margin, thus facilitating operating the switches.

In a preferred aspect of the present invention, the surgical apparatus is characterized in that the vibrating portion is configured so that a high-frequency current to treat the living tissue flows through the vibrating portion and is output.

In the surgical apparatus according to this aspect, the surgical apparatus also functions as a high-frequency surgical apparatus and is frequency used for a long time during an operation, and the effect of the present invention, i.e., facilitating holding and operating the handpiece is prominently demonstrated.

The invention can be more fully understood from the following detailed description when taken in conjunction with the accompanying drawings, in which:
FIG. 1 is a side view showing a surgical instrument according to an embodiment of the present invention;
FIG. 2 is a vertical cross-sectional view showing a handpiece according to an embodiment of the present invention;
FIG. 3 is a vertical cross-sectional view showing a sheath unit according to an embodiment of the present invention; and
FIG. 4 is a partially cross-sectional side view showing a surgical instrument according to a modification of an embodiment of the present invention.

An embodiment according to the present invention will now be explained hereinafter in detail.

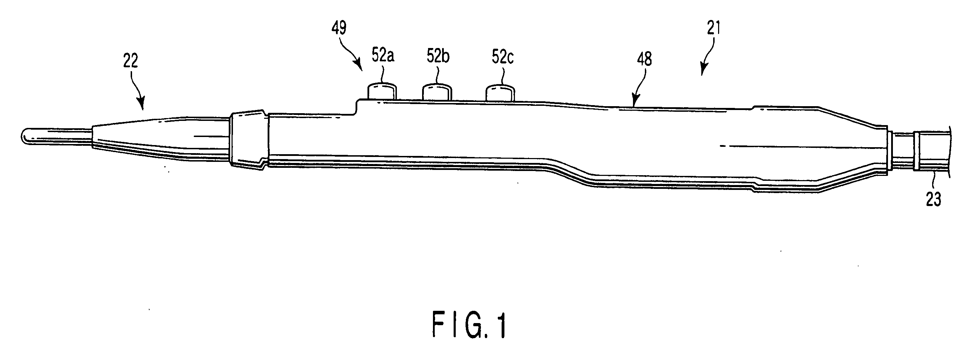

FIGS. 1 to 3 show an embodiment according to the present invention.

A surgical instrument as a surgical treatment apparatus according to this embodiment is an ultrasonic output combined high-frequency surgical instrument for abdominal operations.

Referring to FIG. 1, the surgical instrument has an elongated shape as a whole and is extended in an axial direction. The surgical instrument includes a handpiece 21 to be held and operated by an operator. A sheath unit 22 to treat a living tissue is detachably coupled with a distal-end portion of the handpiece 21. On the other hand, an electrical cable 23 is extended from a proximal-end portion of the handpiece 21, and the electrical cable 23 is connected with a main body to drive the surgical instrument.

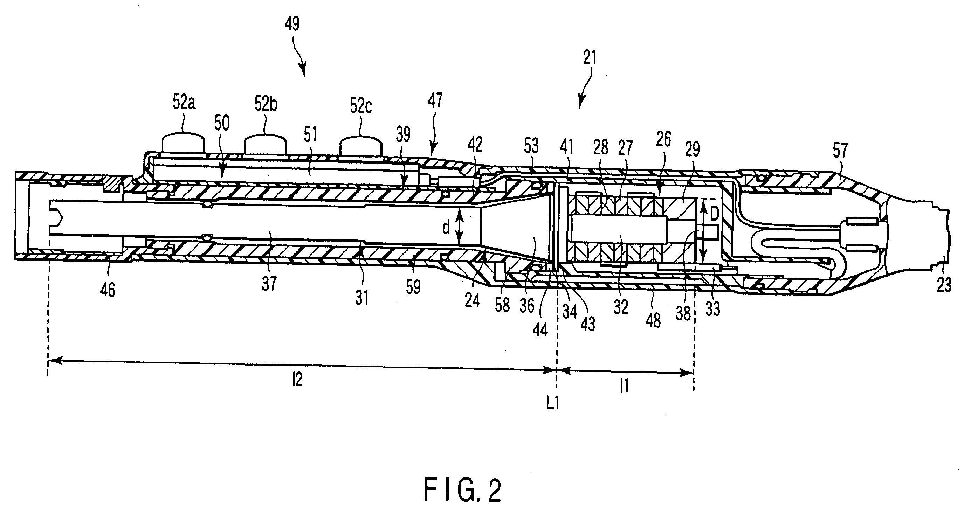

Referring to FIG. 2, the handpiece 21 accommodates a vibrator 24 as a vibrating portion.

A piezoelectric element portion 26 as a vibration generating portion is formed at a proximal-end portion of the vibrator 24. In the piezoelectric element portion 26, a plurality of piezoelectric elements 27 and a plurality of electrodes 28 each having a ring plate shape alternately overlap in the axial direction, and a cylindrical backboard 29 overlaps in the axial direction at a proximal end. The piezoelectric elements 27, the electrodes 28, and the backboard 29 have substantially the same outside diameters, and the piezoelectric element portion 26 has a fixed outside diameter D along the entire axial direction. A proximal-end surface of a horn 31 faces a distal-end surface of the piezoelectric element portion 26. A bolt 32 is protruded from the proximal-end surface of the horn 31 toward the proximal end side in the axial direction. The bolt 32 pierces the piezoelectric elements 27 and the electrodes 28, and the backboard 29 is screwed to a proximal-end portion of the bolt 32. The piezoelectric elements 27 and the electrodes 28 are held by the proximal-end surface of the horn 31 and the backboard 29 through screwing the backboard 29 into the bolt 32. A distal-end portion of an ultrasonic cable 33 for a positive electrode and a negative electrode is connected with a positive electrode and a negative electrode in the plurality of electrodes 28, respectively. The ultrasonic cable 33 is led to the electrical cable 23 and inserted through the electrical cable 23. A driving current is supplied to the piezoelectric element portion 26 from the main body through the ultrasonic cable 33, electrical vibration is converted into mechanical vibration in the piezoelectric element portion 26, thereby producing ultrasonic vibration.

The horn 31 as a vibration transmitting portion has a columnar shape and is extended in the axial direction. A flange portion 34 to fix the horn 31 is formed at a proximal-end portion of the horn 31. An outside diameter of the flange portion 34 is larger than the outside diameter D of the piezoelectric element portion 26. A reduced diameter portion 36 is formed on a distal-end side of the flange portion 34. An outside diameter of a proximal end of the reduced diameter portion 36 matches with the outside diameter D of the piezoelectric element portion 26, and the outside diameter of the reduced diameter portion 36 is reduced from the proximal-end side toward the distal-end side. An extending portion 37 is formed on the distal-end side of the reduced diameter portion 36. An outside diameter of a proximal end of the extending portion 37 matches with the outside diameter of the distal end of the reduced diameter portion 36, and the extending portion 37 has a substantially fixed outside diameter d along the entire axial direction. Therefore, an outside diameter d of the extending portion 37 is smaller than the outside diameter D of the piezoelectric element 27. A distal-end portion of the extending portion 37 is arranged at the distal-end portion of the handpiece 21.

A proximal end and a distal end of the vibrator 24 form antinode positions of the ultrasonic vibration, and an axial length L1 of the vibrator 24 is a length corresponding to one wavelength of the ultrasonic vibration. Moreover, the proximal end of the piezoelectric element portion 26 forms the antinode position of the ultrasonic vibration, the distal end of the piezoelectric element portion 26 forms a node position of the ultrasonic vibration, and an axial length 11 of the piezoelectric element portion 26 is a length corresponding to a quarter wavelength of the ultrasonic vibration. Additionally, the proximal end of the horn 31 forms the node position of the ultrasonic vibration, the distal end of the horn 31 forms the antinode position of the ultrasonic vibration, and an axial length 12 of the horn 31 is a length corresponding to a three-quarter wavelength of the ultrasonic vibration.

Further, a distal-end portion of a high-frequency cable 38 is connected with the negative electrode in the plurality of electrodes 28 in the piezoelectric element portion 26. The high-frequency cable 38 is led to the electrical cable 23 and inserted through the electrical cable 23. A high-frequency current is supplied to the piezoelectric element portion 26 from the main body through the high-frequency cable 38 and flows through the vibrator 24.

The vibrator 24 is accommodated in a cylindrical inner housing 39. The inner housing 39 is coaxially extended with the vibrator 24 in the axial direction. Furthermore, the inner housing 39 is formed of a proximal-end-side inner cylinder 41 and a distal-end-side inner cylinder 42.

The piezoelectric element portion 26 is accommodated in the proximal-end-side inner cylinder 41. The ultrasonic cable 33 extended from the piezoelectric element portion 26 is inserted through an insertion hole formed in the inner housing 39 to be extended to the proximal-end side from the inner housing 39. This is also true with respect to the high-frequency cable 38. A fixing protruding portion 43 is extended in a circumferential direction on an inner peripheral surface of the proximal-end-side inner cylinder 41 on the distal-end side. Moreover, a proximal-end portion of the distal-end-side inner cylinder 42 is fitted and screwed into the distal-end portion of the proximal-end-side inner cylinder 41. The flange portion 34 of the vibrator 24 is held and fixed by the protruding portion 43 of the proximal-end-side inner cylinder 41 and the proximal-end surface of the distal-end-side inner cylinder 42 through screwing the distal-end-side inner cylinder 42 into the proximal-end-side inner cylinder 41. It is to be noted that a spacer 44 to adjust an axial position of the vibrator 24 is interposed between the distal-end surface of the flange portion 34 and the proximal-end surface of the distal-end-side inner cylinder 42. In this manner, the vibrator 24 is fixed to the inner housing 39 at the flange portion 34 serving as a node position of the ultrasonic vibration.

The horn 31 is accommodated in the distal-end-side inner cylinder 42. An inside diameter of the distal-end-side inner cylinder 42 is slightly larger than the outside diameter of the horn 31, and in the distal-end-side inner cylinder 42 are formed a large-diameter portion 58 on the proximal-end side where the reduced diameter portion 36 of the horn 31 is accommodated and a small-diameter portion 59 on the distal-end side where the extending portion 37 of the horn 31 is accommodated. A proximal-end portion of a cylindrical coupling cylinder 46 is coaxially coupled with the distal-end portion of the distal-end-side inner cylinder 42.

The inner housing 39 is accommodated in an outer housing 47. The outer housing 47 is coaxially extended with the inner housing 39 in the axial direction. A proximal-end side of the outer housing 47 forms a support portion 48 to be supported by an operator. On the other hand, a hand switch portion 49 as an operating portion to operate the main body is arranged on a distal-end side of the outer housing 47.

In the inner housing 39 accommodated in the outer housing 47, an outside diameter of the small-diameter portion 59 of the distal-end-side inner cylinder 42 is smaller than outside diameters of the proximal-end-side inner cylinder 41 and the large-diameter portion 58 of the distal-end-side inner cylinder 42. Therefore, an accommodation space 50 is formed in the outer housing 47 on the radially outer side of the small-diameter portion 59. A switch main body 51 of the hand switch portion 49 is accommodated in the accommodation space 50. In the handpiece 21, an outside diameter of the hand switch portion 49 is substantially equal to an outside diameter of the support portion 48, and the outside diameter of the handpiece 21 is substantially constant along the entire axial direction. Hand switches 52a, 52b, and 52c are retractably protruded on the radially outer side of the switch main body 51 toward the outside in the radial direction, and are protruded from the outer housing 47. In this embodiment, the three hand switches 52a, 52b, and 52c are aligned in the axial direction from the distal-end side toward the proximal-end side in the hand switch portion 49. A switch cable 53 is extended from a proximal-end portion of the switch main body 51. The switch cable 53 is inserted through a space between the outer housing 47 and the inner housing 39 to be extended toward the proximal-end side.

A distal-end portion of a proximal-end housing 57 is coaxially coupled with the proximal-end portion of the outer housing 47. A distal-end portion of the electrical cable 23 is connected with a proximal-end portion of the proximal-end housing 57. The ultrasonic cable 33, the high-frequency cable 38, and the switch cable 53 extended from the proximal-end portion of the inner housing 39 are led into the proximal-end housing 57, and then led into the electrical cable 23.

It is to be noted that, in the handpiece 21, a sealing member, e.g., an O-ring is appropriately arranged between respective members to keep the inside fluid-tight and protect electrical elements and others, and therefore the handpiece 21 can be treated by autoclave sterilization using high-temperature highpressure water vapor.

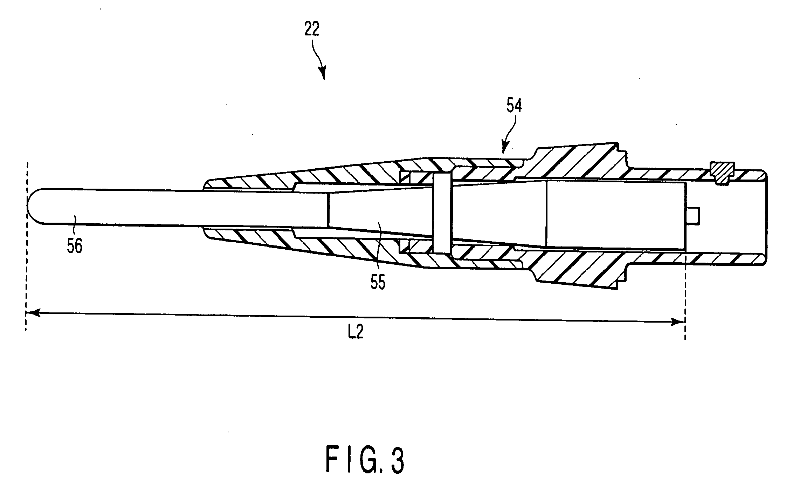

Referring to FIG. 3, the sheath unit 22 detachably disposed to the handpiece 21 includes a cylindrical sheath 54. A columnar probe 55 is fitted into the sheath 54, and the probe 55 is held by the sheath 54. A distal-end portion of the probe 55 is protruded from a distal-end portion of the sheath to form a treatment portion 56 to treat a living tissue.

Referring to FIGS. 1 to 3, a coupling mechanism to couple the sheath unit 22 with the handpiece 21 detachably and coaxially is formed at the coupling cylinder 46 of the handpiece 21 and the proximal-end portion of the sheath 54 in the sheath unit 22. When the sheath unit 22 is coupled with the handpiece 21, the proximal-end portion of the probe 55 of the sheath unit 22 is pressed against the distal-end portion of the horn 31 of the handpiece 21. The vibrator 24 of the handpiece 21 and the probe 55 of the sheath unit 22 are ultrasonic-vibrated integrally, the proximal end and the distal end of the probe 55 serve as antinode positions of the vibration, and an axial length L2 of the probe 55 is a length corresponding to a half wavelength of the ultrasonic vibration. Further, a high-frequency current flows through the vibrator 24 of the handpiece 21 and so the high-frequency current flows through the probe 55.

As explained above, in the surgical instrument, a high-frequency current is output to the piezoelectric element portion 26, the high-frequency current flows through the vibrator 24 and the probe 55, and the treatment portion 56 of the probe 55 is press against a living tissue, thereby giving the living tissue a high-frequency treatment. Furthermore, a driving current is output to the piezoelectric element portion 26, the vibrator 24 and the probe 55 are ultrasonic vibrated integrally, and the treatment portion 56 of the probe 55 is pressed against a living tissue, thereby giving the living tissue an ultrasonic treatment. Moreover, the surgical instrument can operate in various modes in accordance with a high-frequency current and a driving current output to the piezoelectric element portion 26. For example, a high-frequency incision mode where strong incision and very weak coagulation are performed by means of the high-frequency current; a high-frequency coagulation mode where strong coagulation and very weak incision are performed by means of the high-frequency current; a simultaneously incision mode where strong incision and weak coagulation are performed by means of both the high-frequency current and the ultrasonic vibration; a simultaneous coagulation mode where strong coagulation and strong incision are performed by means of both the high-frequency current and the ultrasonic vibration; and an ultrasonic incision and dissection mode where weak coagulation and weak incision are performed by means of the ultrasonic vibration. In the surgical instrument according to this embodiment, three modes are adopted, and when any one of the three hand switches 52a, 52b, and 52c is pressed, the surgical instrument is activated in any one of the three modes. A mode to be adopted and assignment of the modes to the hand switches 52a, 52b, and 52c may be arbitrarily set. For example, (i) the high-frequency incision mode/the high-frequency coagulation mode/the simultaneous coagulation mode, (ii) the high-frequency incision mode/the simultaneous coagulation mode/the high-frequency coagulation mode, (iii) the high-frequency incision mode/the simultaneous coagulation mode/the ultrasonic incision and dissection mode, (iv) the high-frequency incision mode/the ultrasonic incision and dissection mode/the simultaneous coagulation mode, (v) the high-frequency incision mode/the high-frequency coagulation mode/the ultrasonic incision and dissection mode, or (vi) the simultaneous incision mode/the simultaneous coagulation mode/the ultrasonic incision and dissection mode may be assigned to the hand switches 52a, 52b, and 52c at the distal end/middle/proximal end position. It is to be noted that the hand switch for incision is provided closer to the distal-end side than the hand switch for coagulation on medical standards.

In the surgical instrument according to this embodiment, the axial length L1 of the vibrator 24 is the length corresponding to one wavelength or above of the ultrasonic vibration. Therefore, an axial length of the handpiece 21 is a length corresponding to approximately one wavelength or above of the ultrasonic vibration, thereby securing the axial length that facilitates holding and operating. In this embodiment, in particular, since the axial length L1 of the vibrator 24 is the length corresponding to one wavelength of the ultrasonic vibration and an axial length of the handpiece 21 is a length corresponding to approximately one wavelength, this is the length suitable for holding and operating the handpiece in one hand. Specifically, the axial length 11 of the piezoelectric element portion 26 of the vibrator 24 is the length corresponding to the quarter wavelength of the ultrasonic vibration, and an axial length of the support portion 48 to be supported by an operator is a length corresponding to approximately quarter wavelength of the ultrasonic vibration. Additionally, the axial length 12 of the horn 31 of the vibrator 24 is the length corresponding to the three-quarter wavelength or above of the ultrasonic vibration, and the hand switch portion 49 is arranged along the extending portion 37 of the horn 31. With such a structure, an arrangement and a shape are secured, the arrangement and the shape suitable for operating the hand switch portion 49 by a finger of a hand holding the handpiece 21 while fitting the support portion 48 at a base portion of a thumb and an index finger when an operator holds the handpiece 21. Although the two or more hand switches 52a, 52b, and 52c are aligned in the axial direction in the hand switch portion 49, an axial length of the hand switch portion 49 is a length corresponding to approximately a three-quarter wavelength or above of the ultrasonic vibration, and hence the two or more hand switches 52a, 52b, and 52c can be arranged with an appropriate margin. In particular, when the three hand switches 52a, 52b, and 52c are aligned and arranged in the axial direction like this embodiment, securing a length corresponding to a three-quarter wavelength of the ultrasonic vibration as the axial length 12 of the horn 31 of the vibrator 24 realizes a preferable arrangement. In addition, the outside diameter d of the extending portion 37 of the horn 31 is smaller than the outside diameter D of the piezoelectric element portion 26, and so the outside diameter of the small-diameter portion 59 of the distal-end-side inner cylinder 42 accommodating the extending portion 37 of the horn 31 is smaller than the outside diameter of the proximal-end-side inner cylinder 41 accommodating the piezoelectric element portion 26, and the switch main body 51 of the hand switch portion 49 is accommodated in the accommodation space 50 on the radially outer side of the small-diameter portion 59. Therefore, in the hand piece 21, the outside diameter of the hand switch portion 49 is equal to or smaller than the outside diameter of the support portion 48, a diameter of the periphery of the hand switch portion 49 is reduced, and so the handpiece 21 can be readily held and operated with respect to an entire balance.

When using the surgical treatment apparatus, the sheath unit 22 is attached to the handpiece 21. After opening an abdomen, the handpiece 21 is held by one hand, the hand switches 52a, 52b, and 52c are pressed to activate the handpiece 21 in various modes, and the handpiece 21 is moved to press the treatment portion 56 of the probe 56 against a living tissue, thereby treating the living tissue. Here, since a high-frequency treatment is often performed for a long time during an operation, an inconvenience occurs when the handpiece 21 is hard to be held and operated. However, since the handpiece 21 can be easily held and operated in this embodiment, an operation can be smoothly carried out.

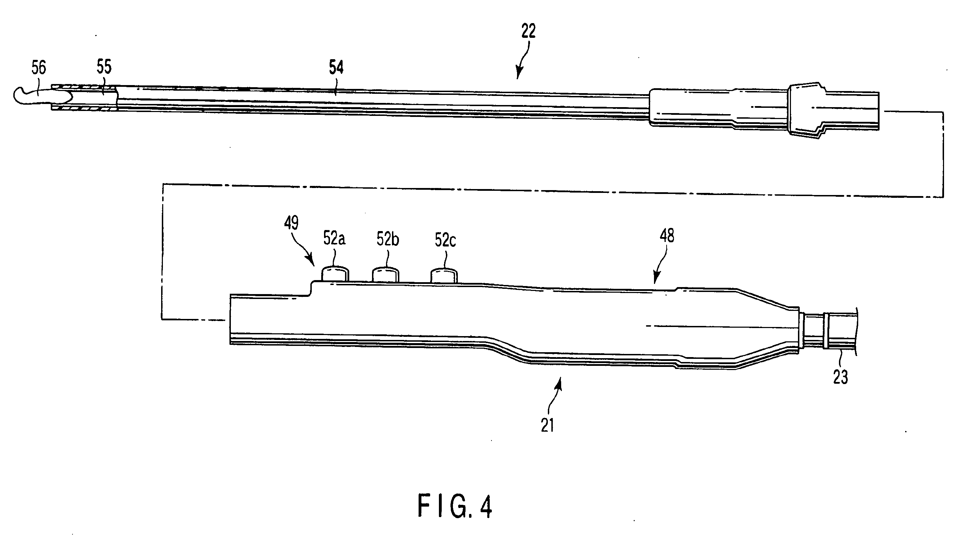

The present invention can be applied to various surgical treatment apparatuses other than the above-explained ultrasonic output combined high-frequency surgical instrument for abdominal operations. For example, the present invention can be also applied to such an ultrasonic output combined high-frequency surgical instrument for endoscopic operations as depicted in FIG. 4. In the surgical instrument depicted in FIG. 4, a handpiece 21 and a sheath unit 22 having the same structures as those in the foregoing embodiment are used. However, a sheath 54 and a probe 55 in the sheath unit 22 are long, the probe 55 has a length which is an integral multiple of a half wavelength of ultrasonic vibration, and a treatment portion 56 has a hook-like shape. Besides, the present invention can be also applied to a surgical instrument in which a sheath unit is undetachably coupled with a handpiece and an ultrasonic surgical instrument having no high-frequency treatment function.

## Claims

1. A handpiece (21) for a surgical treatment apparatus, configured to be held and operated by an operator and extended in an axial direction, the handpiece (21) being further configured to be electrically connected to a main body for driving the handpiece (21), wherein the handpiece (21) includes:
a vibrating portion (24) extended in the axial direction, including a distal end portion and a proximal end portion, wherein the vibrating portion (24) includes:
a vibration generating portion (26) formed on a proximal-end side of the vibrating portion (24) and configured to be supplied with a driving current by the main body to generate ultrasonic vibration having a predetermined wavelength; and
a vibration transmitting portion (31) configured to transmit the ultrasonic vibration generated by the vibration generating portion (26) and being formed on a distal-end side of the vibrating portion (24), wherein the vibration transmitting portion (31) includes: a reduced diameter portion (36) arranged on the proximal-end side of the vibration transmitting portion (31) and having an outside diameter reduced from a proximal-end side toward a distal-end side; and an extending portion (37) arranged on the distal-end side of the vibration transmitting portion (31) and including an outside diameter (d) smaller than an outside diameter (D) of the vibration generating portion (26); and
an operating portion (49) configured to operate the main body to supply the vibration generating portion (26) with the driving current, wherein the operating portion (49) is arranged in an accommodation space (50) formed on a radially outer side of the extending portion (37);
**characterized in that**
the vibrating portion (24) has an axial length being a length corresponding to the predetermined wavelength;
the vibration generating portion (26) has an axial length (11) being a length corresponding to a quarter of the predetermined wavelength; and
the vibration transmitting portion (31) has an axial length (12) being a length corresponding to three-quarters of the predetermined wavelength.

2. The handpiece according to claim 1, **characterized in that** the operating portion (49) includes three switches aligned in the axial direction.

3. The handpiece according to claim 1, **characterized in that**
the operating portion (49) is configured to operate the main body to supply the vibrating portion (24) with high-frequency current; and
the vibrating portion (24) is configured to output the supplied high-frequency current.

4. A surgical treatment apparatus comprising:
the handpiece (21) according to one of claims 1 to 3; and
a sheath unit (22) configured for treating a living tissue and configured to be connected to the handpiece (21).

5. The surgical treatment apparatus according to claim 4, wherein
the sheath unit (22) includes a column or probe (55), and
the column or probe (55) extends in the axial direction, includes a proximal end portion configured to be connected to the distal end portion of the vibrating portion (24) and a distal end portion configured to treat a living tissue, the column or probe (55) being further configured to ultrasonically vibrate by the ultrasonic vibration output by the vibrating portion (24), and having an axial length corresponding to one half of the predetermined wavelength.

## Patentansprüche

1. Handgriffteil (21) für ein chirurgisches Behandlungsinstrument, das dazu eingerichtet ist, von einem Anwender gehalten und bedient zu werden und das sich in eine axiale Richtung erstreckt, wobei das Handgriffteil (21) weiterhin dazu eingerichtet ist, zum Antreiben des Handgriffteils (21), elektrisch mit einem Hauptkörper verbunden zu werden, wobei das Handgriffteil (21) umfasst:
einen schwingenden Abschnitt (24), der sich in der axialen Richtung erstreckt und der einen distalen Endbereich und einen proximalen Endbereich umfasst, wobei der schwingende Abschnitt (24) umfasst:
einen Schwingungserzeugungsabschnitt (26), der an einer proximalen Endseite des schwingenden Abschnitts (24) ausgebildet und dazu eingerichtet ist, durch den Hauptkörper mit einem Antriebsstrom versorgt zu werden, um eine Ultraschallschwingung mit einer vorbestimmten Wellenlänge zu erzeugen; und
einen Schwingungsübertragungsabschnitt (31), der dazu eingerichtet ist, die durch den Schwingungserzeugungsabschnitt (26) erzeugte Ultraschallschwingung zu übertragen und an einer distalen Endseite des schwingenden Abschnitts (24) ausgebildet ist, wobei der Schwingungsübertragungsabschnitt (31) umfasst: einen Abschnitt (36) mit einem reduzierten Durchmesser, der an der proximalen Endseite des Schwingungsübertragungsabschnitts (31) angeordnet ist und einen Außendurchmesser aufweist, der sich von einer proximalen Endseite zu einer distalen Endseite verringert; und einen sich erstreckenden Abschnitt (37), der an der distalen Endseite des Schwingungsübertragungsabschnitts (31) angeordnet ist und einen Außendurchmesser (d) hat, der kleiner als ein Außendurchmesser (D) des Schwingungserzeugungsabschnitts (26) ist; und
einen Betriebsabschnitt (49), der dazu eingerichtet ist, den Hauptkörper zu betreiben, um den Schwingungserzeugungsabschnitt (26) mit dem Antriebsstrom zu versorgen, wobei der Betriebsabschnitt (49) in einem Aufnahmebereich (50) angeordnet ist, der an einer radialen Außenfläche des sich erstreckenden Abschnitts (37) angeordnet ist;
**dadurch gekennzeichnet, dass**
der schwingende Abschnitt (24) eine axiale Länge aufweist, die eine der vorbestimmten Wellenlänge entsprechende Länge ist;
der Schwingungserzeugungsabschnitt (26) eine axiale Länge (11) aufweist, die eine einem Viertel der vorbestimmten Wellenlänge entsprechende Länge ist; und
der Schwingungsübertragungsabschnitt (31) eine axiale Länge (12) aufweist, die eine drei Viertel der vorbestimmten Wellenlänge entsprechende Länge ist.

2. Handgriffteil gemäß Anspruch 1, **dadurch gekennzeichnet, dass** der Betriebsabschnitt (49) drei Schalter umfasst, die in der axialen Richtung ausgerichtet sind.

3. Handgriffteil gemäß Anspruch 1, **dadurch gekennzeichnet, dass**
der Betriebsabschnitt (49) dazu eingerichtet ist, den Hauptkörper zu betreiben, um den schwingenden Abschnitt (24) mit Hochfrequenzstrom zu versorgen; und
der schwingende Abschnitt (24) dazu eingerichtet ist, den bereitgestellten Hochfrequenzstrom auszugeben.

4. Chirurgisches Behandlungsinstrument umfassend:
das Handgriffteil (21) gemäß einem der Ansprüche 1 bis 3; und
eine Hülleneinheit (22) die dazu eingerichtet ist, lebendes Gewebe zu behandeln und die dazu eingerichtet ist, mit dem Handgriffteil (21) verbunden zu werden.

5. Chirurgisches Behandlungsinstrument gemäß Anspruch 4, wobei
die Hülleneinheit (22) eine säulenförmige Sonde (55) umfasst, und
die säulenförmige Sonde (55) sich in der axialen Richtung erstreckt, einen proximalen Endbereich, der dazu eingerichtet ist, mit dem distalen Endbereich des schwingenden Abschnitts (24) verbunden zu werden, und einen distalen Endbereich umfasst, der dazu eingerichtet ist, lebendes Gewebe zu behandeln, wobei die säulenförmige Sonde (55) ferner dazu eingerichtet ist, durch die Ultraschallschwingungsausgabe des schwingenden Abschnitts (24) im Ultraschallbereich zu schwingen, und eine axiale Länge hat, die der Hälfte der vorbestimmten Wellenlänge entspricht.

## Revendications

1. Pièce à main (21) pour un appareil de traitement chirurgical, configurée pour être tenue et opérée par un opérateur et étendue dans un sens axial, la pièce à main (21) étant en outre configurée pour être électriquement connectée à un corps principal pour piloter la pièce à main (21), dans laquelle la pièce à main (21) inclut :
une partie vibrante (24) étendue dans le sens axial, incluant une partie d'extrémité distale et une partie d'extrémité proximale, dans laquelle la partie vibrante (24) inclut :
une partie de génération de vibration (26) formée sur un côté d'extrémité proximale de la partie vibrante (24) et configurée pour être alimentée avec un courant de pilotage par le corps principal pour générer une vibration ultrasonore ayant une longueur d'onde prédéterminée ; et
une partie de transmission de vibration (31) configurée pour transmettre la vibration ultrasonore générée par la partie de génération de vibration (26) et étant formée sur un côté d'extrémité distale de la partie vibrante (24), dans laquelle
la partie de transmission de vibration (31) inclut : une partie de diamètre réduit (36) agencée sur le côté d'extrémité proximale de la partie de transmission de vibration (31) et ayant un diamètre extérieur réduit d'un côté d'extrémité proximale vers un côté d'extrémité distale ; et une partie d'extension (37) agencée sur le côté d'extrémité distale de la partie de transmission de vibration (31) et incluant un diamètre extérieur (d) plus petit qu'un diamètre extérieur (D) de la partie de génération de vibration (26) ; et
une partie opérationnelle (49) configurée pour opérer le corps principal pour alimenter la partie de génération de vibration (26) avec le courant de pilotage, dans laquelle la partie opérationnelle (49) est agencée dans un espace de réception (50) formé sur un côté radialement extérieur de la partie d'extension (37) ;
**caractérisée en ce que**
la partie vibrante (24) a une longueur axiale étant une longueur correspondant à la longueur d'onde prédéterminée ;
la partie de génération de vibration (26) a une longueur axiale (11) étant une longueur correspondant à un quart de la longueur d'onde prédéterminée ; et
la partie de transmission de vibration (31) a une longueur axiale (12) étant une longueur correspondant à trois quarts de la longueur d'onde prédéterminée.

2. Pièce à main selon la revendication 1, **caractérisée en ce que** la partie opérationnelle (49) inclut trois commutateurs alignés dans le sens axial.

3. Pièce à main selon la revendication 1, **caractérisée en ce que**
la partie opérationnelle (49) est configurée pour opérer le corps principal pour alimenter la partie vibrante (24) avec un courant haute fréquence ; et
la partie vibrante (24) est configurée pour délivrer en sortie le courant haute fréquence alimenté.

4. Appareil de traitement chirurgical comprenant :
la pièce à main (21) selon l'une des revendications 1 à 3 ; et
une unité de gaine (22) configurée pour traiter un tissu vivant et configurée pour être connectée à la pièce à main (21).

5. Appareil de traitement chirurgical selon la revendication 4, dans lequel
l'unité de gaine (22) inclut une sonde colonnaire (55), et
la sonde colonnaire (55) s'étend dans le sens axial, inclut une partie d'extrémité proximale configurée pour être connectée à la partie d'extrémité distale de la partie vibrante (24) et une partie d'extrémité distale configurée pour traiter un tissu vivant, la sonde colonnaire (55) étant en outre configurée pour être mise en vibration ultrasonore par la vibration ultrasonore délivrée en sortie par la partie vibrante (24), et ayant une longueur axiale correspondant à la moitié de la longueur d'onde prédéterminée.
